# FASCICULE DE BREVET EUROPEEN

(11) **EP 0 906 101 B1**
(45) Date de publication et mention de la délivrance du brevet: **19.06.2002**
(21) Numéro de dépôt: 97907145.3
(22) Date de dépôt: 28.02.1997
(51) Int. Cl.: A61K 31/495, A61K 9/20

(54) **FORMULATIONS PHARMACEUTIQUES A LIBERATION PROLONGEE CONTENANT DE LA MIZOLASTINE**
ARZNEIMITTEL ENTHALTEND MIZOLASTIN MIT VERZÖGERTER WIRKSTOFFABGABE
SLOW-RELEASE PHARMACEUTICAL FORMULATIONS CONTAINING MIZOLASTIN

(30) Priorité: 04.03.1996 FR 9602662
(43) Date de publication de la demande: 07.04.1999
(73) Titulaire: SANOFI-SYNTHELABO, 75013 Paris (FR)
(72) Inventeur: CHARIOT, Maryvonne, F-91620 La Ville-du-Bois (FR); LEWIS, Gareth, F-91410 Dourdan (FR); MONTEL, Jean, F-78400 Chatou (FR)
(74) Mandataire: Thouret-Lemaitre, Elisabeth
(86) Numéro de dépôt international: FR9700355
(87) Numéro de publication internationale: WO9732584

(56) Documents cités:
- CHEMICAL ABSTRACTS, vol. 107, no. 1, 6 Juillet 1987 Columbus, Ohio, US; abstract no. 7211, XP002018368 cité dans la demande & JP 62 061 979 A (SYNTHELABO S.A.,FR) 18 Mars 1987

## Description

La présente invention a pour objet de nouvelles formulations pharmaceutiques à libération prolongée contenant comme principe actif la 2-[[1-[1-[(4-fluorophényl)méthyl]-1*H*-benzimidazol-2-yl]pipéridin-4-yl]méthylamino]pyrimidin-4-ol ou 2-[[1-[1-[(4-fluorophényl)méthyl]-1*H*-benzimidazol-2-yl]pipéridin-4-yl]méthylamino]pyrimidine-4(1*H*)-one, ou mizolastine.

La mizolastine est décrite dans le brevet européen EP 0217700.
La mizolastine se lie au récepteur histaminique H₁ et inhibe la dégranulation des mastocytes in vitro et in vivo. Elle peut donc être utilisée pour le traitement des allergies respiratoires, cutanées ou oculaires et des manifestations allergiques diverses.

Lors de l'administration par voie orale de formulations à libération immédiate contenant de la mizolastine, on a constaté des effets sédatifs indésirables liés à l'existence d'un pic plasmatique élevé.
C'est pourquoi il était nécessaire de trouver des formulations pour une administration par voie orale qui présentent un profil de libération du principe actif tel qu'il permet d'obtenir un pic plasmatique moins élevé sans diminuer la biodisponibilité.

La demanderesse a basé sa recherche de telles formulations sur l'étude de cinétiques de dissolution de la mizolastine. En effet la mizolastine est une base faible (pK 5,6) peu soluble dans l'eau (13 mg/l à pH neutre) mais beaucoup plus soluble à pH acide (11 g/l à pH 3) ; les premières gélules libéraient 100 % de mizolastine en 30 minutes dans un milieu de dissolution à pH 2 alors que seulement 40 % étaient dissous à pH 6,8.
En outre, la libération de la mizolastine à partir de la forme pharmaceutique à libération prolongée selon l'invention ne devait pas être influencée par les différences de pH au sein du tractus gastro-intestinal.
Le but de la présente invention est de proposer des formulations contenant de la mizolastine dont le profil de dissolution est le suivant :
- environ 30 à 70 % de mizolastine dissous en 1 heure,
- 100 % de mizolastine dissous en 3 à 5 heures et
- profil indépendant du pH.

La demanderesse a montré que des comprimés comportant un noyau formé d'un comprimé à libération prolongée comportant de la mizolastine associée à une matrice grasse et à un acide organique, le dit comprimé étant enrobé pour éviter la dégradation du produit à la lumière, conviennent parfaitement.

Les comprimés selon l'invention contiennent de 1 mg à 25 mg de mizolastine. Ces doses correspondent à des concentrations de 0,5 % à 12 % en poids de mizolastine.

La matrice grasse est réalisée avec de l'huile de ricin hydrogénée ou avec des lécithines hydrogénées ou des acides gras à longue chaîne, par exemple en C₁₂-C₂₈, tels que l'acide béhénique ou des triglycérides estérifiés avec des acides gras à chaîne moyenne, par exemple des acides gras en C₈-C₁₈.

L'acide organique dont le pK est de préférence égal ou supérieur à 2, est choisi parmi les acides maléïque, tartrique, malique, fumarique, lactique, citrique, adipique succinique sous forme de racémates ou d'isomères. Selon l'invention, l'acide particulièrement préféré est l'acide L-tartrique.

Le rapport en poids entre la mizolastine et l'acide organique doit être compris entre 0,3 et 1. Avec l'acide L-tartrique ce rapport est de préférence égal à 0,5.

Les comprimés sont préparés par granulation à partir du principe actif, de l'agent constitutif de la matrice grasse, de l'acide organique et d'autres excipients tels que par exemple le lactose, le mannitol et les sucres ou sucres-alcools analogues, la cellulose microcristalline, l'amidon, les phosphates et sulfates de calcium, la polyvidone, les celluloses substituées comme l'hydroxypropylcellulose, l'hydroxypropylméthylcellulose ou la méthylcellulose.

La granulation peut être effectuée en phase humide par exemple en présence d'eau ou d'alcool ou réalisée par fusion ou par compactage. L'étape de granulation peut éventuellement être supprimée et les comprimés réalisés par compression directe du mélange de la mizolastine et des excipients.

La silice colloïdale anhydre et le stéarate de magnésium sont ajoutés aux granulés obtenus et le mélange est comprimé.
Les comprimés sont ensuite entourés d'un film d'enrobage par pulvérisation d'une solution d'enrobage dans un appareil à lit d'air fluidisé ou en turbine d'enrobage.

L'exemple suivant illustre l'invention sans la limiter :

| Comprimé | % (poids) |
|---|---|
| mizolastine | 4,8 |
| Huile de ricin hydrogénée | 12,0 |
| Lactose | 60,0 |
| Cellulose microcristalline | 9,6 |
| Acide *L*-tartrique | 9,6 |
| Polyvidone | 2,9 |
| Silice colloïdale anhydre | 0,2 |
| Stéarate de magnésium | 0,9 |
| Eau purifiée | Q.S. |
| **Total** | **100,0** |

| Enrobage | |
|---|---|
| Méthylhydroxypropylcellulose | 74,0 |
| Dioxyde de titane (E171) | 18,5 |
| Propylène glycol | 7,5 |
| Eau purifiée | Q.S. |
| **Total** | **100,0** |

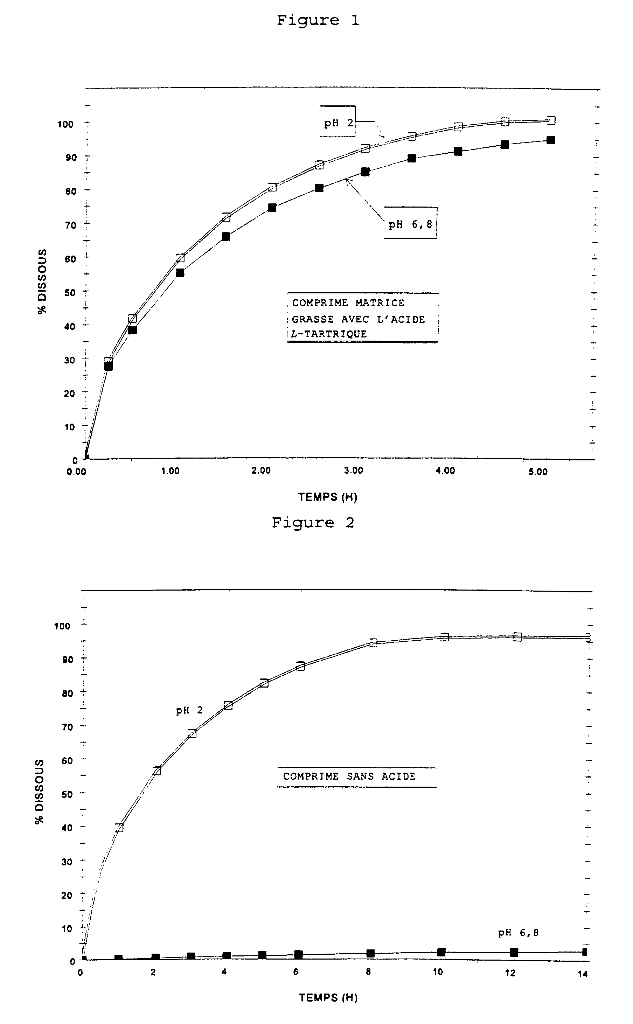

Le profil de dissolution obtenu avec une formulation selon l'invention est donné dans la figure 1.
Ce profil donne environ 50 % de produit dissous en 1 heure, 100 % de produit dissous en 3 à 5 heures et il est indépendant du pH.
Le profil de dissolution obtenu avec une formulation identique à celle de l'invention mais ne contenant pas d'acide *L*-tartrique est donné dans la figure 2.

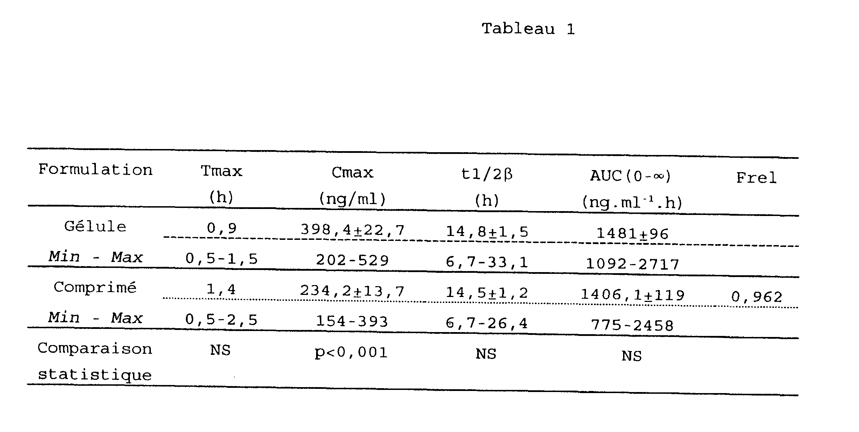

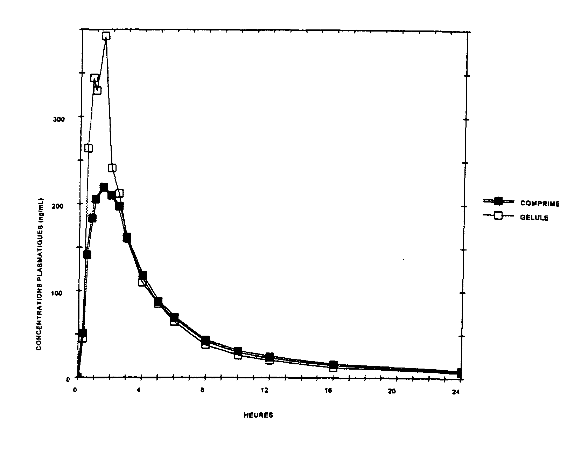

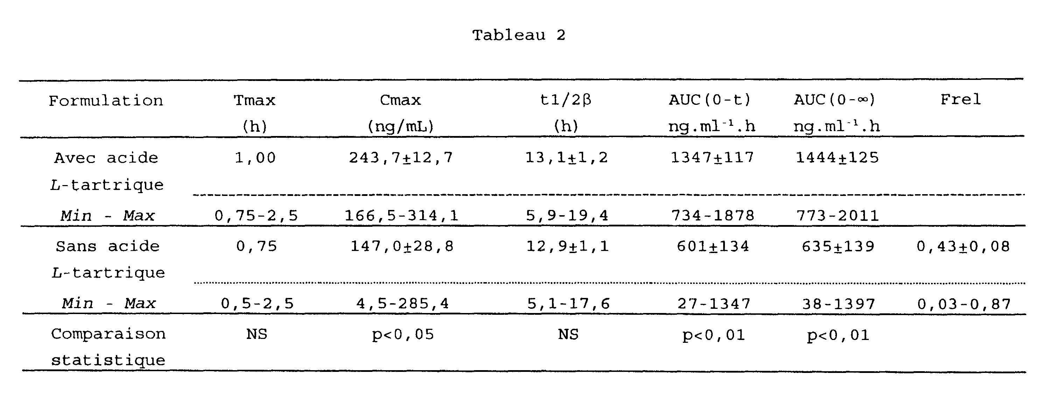

La cinétique plasmatique d'une forme pharmaceutique selon l'invention contenant 10 mg de mizolastine a été étudiée chez le volontaire sain après une administration unique par voie orale, comparativement à une gélule standard à libération immédiate contenant 10 mg de mizolastine.
Le tableau 1 présente les paramètres cinétiques et la figure 3 les courbes des cinétiques plasmatiques, obtenus respectivement avec chaque formulation ; la cinétique plasmatique obtenue avec la forme pharmaceutique selon l'invention permet d'éviter tout pic plasmatique sans perdre de biodisponibilité.

La cinétique plasmatique d'une forme pharmaceutique selon l'invention a également été étudiée en comparaison avec la même formulation sans acide *L*-tartrique.
L'étude a été réalisée chez douze volontaires sains après une administration unique par voie orale d'un comprimé selon l'invention contenant 10 mg de mizolastine ou du même comprimé sans acide *L*-tartrique.
Le tableau 2 montre que la biodisponibilité de la formulation ne contenant pas l'acide *L*-tartrique représente seulement 43 % de celle observée avec la formulation selon l'invention contenant l'acide *L*-tartrique. Les valeurs de Cmax et les AUC (0-∞) sont respectivement 1,5 et 2 fois plus élevées pour la formulation contenant l'acide *L*-tartrique que pour celle n'en contenant pas.
En outre pour la formulation avec l'acide *L*-tartrique, les index de variation mini-maxi sont beaucoup plus faibles, ce qui suggère une grande régularité dans la libération.

L'ensemble des résultats montre que les formulations selon l'invention présentent :
- un profil de dissolution indépendant du pH,
- une libération *in vivo* qui évite tout pic plasmatique,
- une biodisponibilité qui n'est pas diminuée par rapport à une formulation à libération immmédiate,
- une plus faible variabilité des résultats de cinétique plasmatique.

## Revendications

1. Formulation pharmaceutique à libération prolongée contenant de la mizolastine, **caractérisée en ce qu'**elle comporte un noyau formé d'un comprimé à libération prolongée comportant de la mizolastine associée à une matrice grasse et à un acide organique, le dit comprimé étant enrobé.

2. Formulation pharmaceutique à libération prolongée selon la revendication 1, **caractérisée en ce que** le rapport en poids entre la mizolastine et l'acide organique est compris entre 0,3 et 1.

3. Formulation pharmaceutique à libération prolongée selon l'une quelconque des revendications 1 et 2, **caractérisée en ce que** la matrice grasse est réalisé avec de l'huile de ricin hydrogéné ou avec des lécithines hydrogénées ou des acides gras à longue chaîne ou des triglycérides estérifiés avec des acides gras à chaîne moyenne.

4. Formulation pharmaceutique à libération prolongée selon l'une quelconque des revendications 1 à 3, **caractérisée en ce que** l'acide organique est choisi parmi les acides maléïque, tartrique, malique, fumarique, lactique, citrique, adipique succinique sous forme de racémates ou d'isomères.

5. Formulation pharmaceutique à libération prolongée selon l'une quelconque des revendications 1 à 4, **caractérisée en ce que** l'acide organique est l'acide *L*-tartrique.

6. Formulation pharmaceutique à libération prolongée selon la revendication 5, **caractérisée en ce que** le rapport entre la mizolastine et l'acide *L*-tartrique est 0,5.

7. Formulation selon l'une quelconque des revendications 1 à 6 **caractérisée en ce qu'**elle contient de 1 à 25 mg de mizolastine.

8. Formulation selon l'une quelconque des revendications 1 à 7 **caractérisée en ce que** le comprimé contient :
| | % (poids) |
|---|---|
| mizolastine | 4,8 |
| Huile de ricin hydrogénée | 12,0 |
| Lactose | 60,0 |
| Cellulose microcristalline | 9,6 |
| Acide *L*-tartrique | 9,6 |
| Polyvidone | 2,9 |
| Silice colloïdale anhydre | 0,2 |
| Stéarate de magnésium | 0,9 |
| Eau purifiée | Q.S. |
| **Total** | **100,0,** |
ledit comprimé étant enrobé par:
| | % (poids) |
|---|---|
| Méthylhydroxypropylcellulose | 74,0 |
| Dioxyde de titane (E171) | 18,5 |
| Propylène glycol | 7,5 |
| Eau purifiée | Q.S. |
| **Total** | **100,0.** |

## Patentansprüche

1. Pharmazeutische Zubereitung mit verzögerter Wirkstoffabgabe enthaltend Mizolastin, **dadurch gekennzeichnet, daß** sie einen Kern aus einer Tablette mit verzögerter Wirkstofffreisetzung enthaltend Mizolastin in Kombination mit einer Fettmatrix und einer organischen Säure umfaßt, wobei die Tablette umhüllt ist.

2. Pharmazeutische Zubereitung mit verzögerter Wirkstofffreisetzung nach Anspruch 1, **dadurch gekennzeichnet, daß** das Gewichtsverhältnis von Mizolastin zu organischer Säure zwischen 0,3 und 1 ist.

3. Pharmazeutische Zubereitung mit verzögerter Wirkstofffreisetzung nach einem der Ansprüche 1 und 2, **dadurch gekennzeichnet, daß** die Fettmatrix durch hydriertes Rizinusöl oder durch hydrierte Lecithine oder langkettige Fettsäuren oder mit Fettsäuren mit mittlerer Kettenlänge veresterte Triglyceride gebildet ist.

4. Pharmazeutische Zubereitung mit verzögerter Wirkstofffreisetzung nach einem der Ansprüche 1 bis 3, **dadurch gekennzeichnet, daß** die organische Säure aus Maleinsäure, Weinsäure, Äpfelsäure, Fumarsäure, Milchsäure, Citronensäure, Adipinsäure, Bernsteinsäure in Form der Racemate oder der Isomeren ausgewählt ist.

5. Pharmazeutische Zubereitung mit verzögerter Wirkstofffreisetzung nach einem der Ansprüche 1 bis 4, **dadurch gekennzeichnet, daß** die organische Säure *L*-Weinsäure ist.

6. Pharmazeutische Zubereitung mit verzögerter Wirkstofffreisetzung nach Anspruch 5, **dadurch gekennzeichnet, daß** das Verhältnis von Mizolastin zu *L*-Weinsäure 0,5 beträgt.

7. Zubereitung nach einem der Ansprüche 1 bis 6, **dadurch gekennzeichnet, daß** sie 1 bis 25 mg Mizolastin enthält.

8. Zubereitung nach einem der Ansprüche 1 bis 7, **dadurch gekennzeichnet, daß** die Tablette:
| | Gew.-% |
|---|---|
| Mizolastin | 4,8 |
| hydriertes Rizinusöl | 12,0 |
| Lactose | 60,0 |
| mikrokristalline Cellulose | 9,6 |
| *L*-Weinsäure | 9,6 |
| Polyvidon | 2,9 |
| wasserfreies kolloidales Siliciumdioxid | 0,2 |
| Magnesiumstearat | 0,9 |
| gereinigtes Wasser | soweit erforderlich |
| **Summe** | **100,0** |
enthält, wobei die Tablette umhüllt ist mit:
| | Gew.-% |
|---|---|
| Methylhydroxypropylcellulose | 74,0 |
| Titandioxid (E171) | 18,5 |
| Propylenglykol | 7,5 |
| gereinigtes Wasser | soweit erforderlich |
| **Summe** | **100,0.** |

## Claims

1. Sustained-release pharmaceutical formulation containing mizolastine, **characterized in that** it contains a core formed of a sustained-release tablet containing mizolastine combined with a fatty matrix and with an organic acid, the said tablet being coated.

2. Sustained-release pharmaceutical formulation according to Claim 1, **characterized in that** the weight ratio between the mizolastine and the organic acid is between 0.3 and 1.

3. Sustained-release pharmaceutical formulation according to either of Claims 1 and 2, **characterized in that** the fatty matrix is made with hydrogenated castor oil or with hydrogenated lecithins or long-chain fatty acids or triglycerides esterified with medium-chain fatty acids.

4. Sustained-release pharmaceutical formulation according to any one of Claims 1 to 3, **characterized in that** the organic acid is chosen from maleic, tartaric, malic, fumaric, lactic, citric, adipic and succinic acids in the form of racemates or isomers.

5. Sustained-release pharmaceutical formulation according to any one of Claims 1 to 4, **characterized in that** the organic acid is L-tartaric acid.

6. Sustained-release pharmaceutical formulation according to Claim 5, **characterized in that** the ratio between the mizolastine and the L-tartaric acid is 0.5.

7. Formulation according to any one of Claims 1 to 6, **characterized in that** it contains from 1 to 25 mg of mizolastine.

8. Formulation according to any one of Claims 1 to 7, **characterized in that** the tablet contains:
| | % (weight) |
|---|---|
| mizolastine | 4.8 |
| hydrogenated castor oil | 12.0 |
| lactose | 60.0 |
| microcrystalline cellulose | 9.6 |
| *L*-tartaric acid | 9.6 |
| polyvidone | 2.9 |
| anhydrous colloidal silica | 0.2 |
| magnesium stearate | 0.9 |
| purified water | Q.S. |
| **Total** | **100.0,** |
| the said tablet being coated with: | % (weight) |
|---|---|
| methylhydroxypropylcellulose | 74.0 |
| titanium dioxide (E171) | 18.5 |
| propylene glycol | 7.5 |
| purified water | Q.S. |
| **Total** | **100.0.** |
